# EUROPEAN PATENT APPLICATION

(11) **EP 1 164 188 A1**
(43) Date of publication of application: **19.12.2001**
(21) Application number: 01305216.2
(22) Date of filing: 15.06.2001
(51) Int. Cl.: C12N 1/20, A61K 39/04, A61P 31/06

(54) **Auxotrophic Mycobacterium mutants and tuberculosis vaccine**

(30) Priority: 16.06.2000 GB 0014845
(71) Applicant: GLAXO GROUP LIMITED, Greenford, Middlesex UB6 ONN (GB)
(72) Inventor: Parish, Tanya c/o Dept. Med. Microbiology, London E1 2AA (GB); Stoker, Neil c/o London School of Trop. & Hyg. Med, London WC1E 7HT (GB); Smith, Debbie, London School of Trop. & Hyg. Med, London WC1E 7HT (GB); Bancroft, Greg,London School of Trop. & Hyg. Med, London WC1E 7HT (GB)
(74) Representative: Woods, Geoffrey Corlett

(57) **Abstract**

A mycobacterium attenuated by a mutation in a gene that expresses a product which promotes synthesis of proline or tryptophan for use in a method of vaccinating a host against tuberculosis. The mycobacterium may be *Mycobacterium tuberculosis* or *Mycobacterium bovis.*

## Description

### Field of the invention

The invention relates to a bacterium for use in vaccinating against tuberculosis.

### Background to the invention

Infection with *Mycobacterium tuberculosis* (*M*. *tuberculosis)* continues to be a major cause of morbidity and mortality throughout the world. Currently available drug treatments require a minimum six month course of treatment with a cocktail of drugs to which resistance is increasing. The current vaccine *Mycobacterium bovis* Bacille Calmette Guerin (BCG) provides inconsistent and limited efficacy.

### Summary of the invention

The inventors have found that mutations which disrupt the availability of amino acids to a mycobacterium cause attenuation of the mycobacterium. Mutations in the *proC* and *trpD* genes cause decreased virulence of the mycobacterium in a host. The attenuated mycobacteria were found to stimulate protective immunity against normal growth of *M*. *tuberculosis* after a challenge infection.

Accordingly the invention provides:
- use of a mycobacterium attenuated by a mutation in a gene that expresses a product which promotes synthesis of proline or tryptophan in the manufacture of a medicament for vaccination against tuberculosis.
- a mycobacterium attenuated by a mutation in a gene that expresses a product which promotes synthesis of proline or tryptophan,
- a culture comprising the mycobacterium and a culture medium which comprises the amino acid synthesised by the said gene product,
- a method of making the mycobacterium comprising introducing a mutation into the gene of the mycobacterium which promotes synthesis of the amino acid, and
- a method of replicating the mycobacterium comprising culturing the mycobacterium in a culture medium which comprises the amino acid.

### Brief description of the drawing

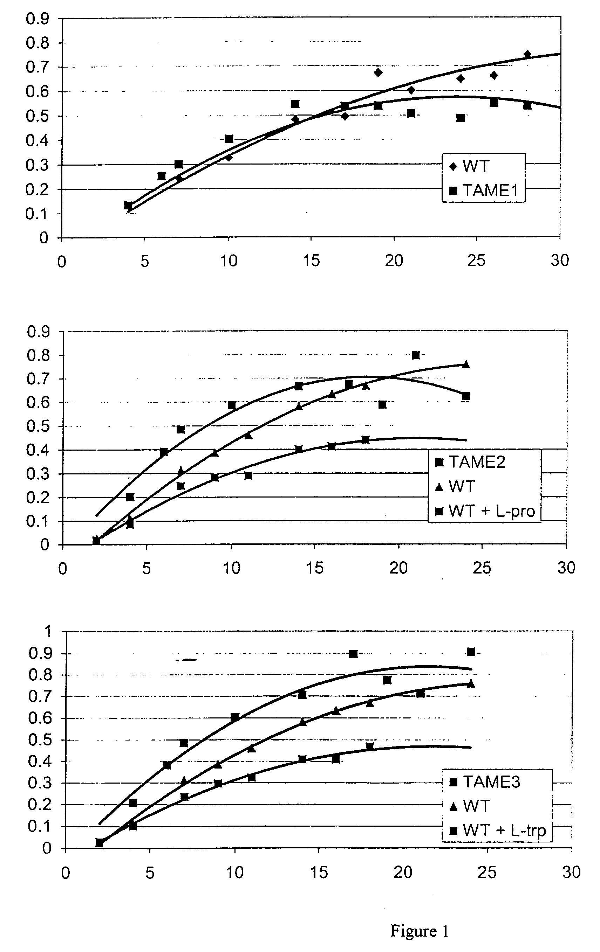

Figure 1 shows the effect of loss of genes involved in amino acid biosynthesis on growth kinetics of *M*. *tuberculosis* in culture. Standing cultures were inoculated with equal numbers of H37Rv or mutants *metB*, *proC* and *trpD* and OD₆₀₀ taken over 0-30 days.

### Description of the Sequence Listing

SEQ ID NO: 1 shows the mutant *trpD* sequence.
SEQ ID NO: 2 shows the mutant *proC* sequence (with hygromycin gene insertion).
SEQ ID NO: 3 shows the mutant *trpD* sequence (with a hygromycin gene insertion).
SEQ ID NO: 4 shows the mutant *proC* sequence.

### Detailed description of the invention

The invention provides a mycobacterium which does not cause disease, but which when administered to a host causes an anti-mycobacterial immune response. The mycobacterium can therefore be used to vaccinate against the disease caused by the mycobacterium.

The host is a human or animal. The animal is generally a mammal, such as a cow, badger, deer or rodent (e.g. a mouse). The human host may be at risk of a mycobacterial infection, typically for socio-economic reasons or may have a genetic or acquired predisposition to mycobacterial infection. Such a predisposition may be caused by an infection with another pathogen (e.g. HIV). The host is typically a neonate or at least 1 year, such as least 5, 10, 20 or more years old.

The mycobacterium of the invention is one which in its wild-type form is generally able to cause or contribute to tuberculosis disease. The mycobacterium is typically *M*. *tuberculosis* or *M. bovis*

The mycobacterium has an attenuated phenotype *in vivo,* i.e. has decreased virulence in the host. Thus when administered to the host the mycobacterium causes reduced, or no, disease symptoms. Thus the levels of mycobacteria found in the host after administration will generally be lower than the levels of wild-type mycobacteria present during disease (strain H37rv is in this case our example of a wild-type mycobacterium). The mycobacterium may have a decreased replication rate in the host (e.g in the macrophages of the host).

Typically the mycobacterium will require one or more additional nutrients to grow in culture, i.e. the mycobacterium will be auxotrophic for such a nutrient (in comparison to the wild-type). Generally the mycobacterium is auxotrophic for the amino acid whose synthesis is affected by the mutation. The mycobacterium may also have a decreased rate of growth/replication when cultured *in vitro* in host cells, such as macrophages.

### The gene in which the attenuating mutation is present

The gene which is mutated is one whose product promotes synthesis of tryptophan or proline. A gene may be mutated whose product promotes synthesis of tryptophan and a gene may be mutated whose product promotes synthesis of proline. The product is typically an enzyme, such as an enzyme in the amino acid biosynthesis pathway. Such an enzyme generally acts on a precursor of one or more amino acids. Thus the gene is typically *trpD* or *proC*. Typically the mycobacterium has both of these specific mutations.

### Second and further mutations

The mycobacterium preferably comprises a mutation in one, two or more genes in addition to the mutation in the gene expressing a product which promotes synthesis of proline or tryptophan. This minimises the risk of the mycobacterium reverting to the virulent state, which is clearly important for the use of the mycobacterium as a vaccine.

Typically the further mutations are in any of the following genes: the *pirC*, *erp*, *pps*, *fadD28*, *mmpL7*, *hspR*, *fadD26*, *pks6*, *Rv2452c*, *Rv1395*, *lipF, drrrC*, *mmpL2*, *Rv0204, modA*, *mmpL4, Rv3081c*, *cmaA*, *narG* or *fbpA* gene.

### The nature of the mutation

The mutations introduced into the mycobacterial gene (including the second and further mutations discussed above) may reduce the function of the gene completely or partially. This is generally achieved either by reducing or abolishing synthesis of a product from the gene or by making a mutation that results in synthesis of a product with reduced or no activity. One such activity may be the ability of the product to promote synthesis of the amino acid.

The mutation may be in a coding or non-coding region of the gene. In order to abolish synthesis of product the entire gene or a part (e.g. 5', 3' or middle portion) is typically deleted. The mutation may be an insertion, but is preferably a substitution or deletion. A mutation in the coding sequence of a gene may be used to create a gene that synthesises a polypeptide with reduced activity (e.g. polypeptide that contains only the N-terminal of the wild-type protein).

The mutations are preferably non-reverting mutations. These are mutations that show essentially no reversion back to the wild-type when the mycobacterium is used as a vaccine. Such mutations include insertions and deletions. Insertions and deletions (and also substitutions) are preferably large, typically at least 10 nucleotides in length, for example from 10 to 1500 nucleotides, such as 500 to 1000 nucleotides.

The mycobacterium preferably contains only defined mutations, i.e. mutations which are characterised. It is clearly undesirable to use a mycobacterium which has uncharacterised mutations in its genome as a vaccine because there would be a risk that the uncharacterised mutations may confer properties on the bacterium that cause undesirable side-effects.

The mycobacterium may or may not be resistant to one or more substances which are used for selection. Such a substance is generally one which kills (or at least inhibits the growth) of the wild-type mycobacterium, for example a suitable antibiotic (e.g hygromycin). Thus the mycobacterium may or may not comprise a gene which expresses a product which causes or promotes such resistance.

The attenuating mutation may be constructed by methods well known to those skilled in the art, such as transposon based techniques. One method for introducing a mutation into the gene is based on the use of homologous recombination. Typically such a method comprises contacting the genome of the mycobacterium with a polynucleotide which is able to recombine with a region of the genome that includes all or part of the gene. The sequence of such a polynucleotide generally corresponds to the sequence of the relevant genome region (i.e. the same or homologous) apart from a deletion, insertion or substitution (compared to the genome region) which corresponds to the desired mutation. Allowing recombination between the polynucleotide and the region causes the mutation to be introduced into the genome region.

### Expression of heterologous antigens

The mycobacterium may be genetically engineered to express an antigen from another organism (a 'heterologous antigen'), so that the attenuated bacterium acts as a carrier of the antigen from the other organism. In this way vaccination with the mycobacterium can be used to provide protection against the other organism. A multivalent vaccine may be produced which not only provides immunity against the virulent parent of the attenuated mycobacterium but also provides immunity against the other organism. Furthermore, the mycobacterium may be engineered to express more than one heterologous antigen, in which case the heterologous antigens may be from the same or different organisms.

The heterologous antigen may be a complete protein or a part of a protein containing an epitope. The antigen may be a bacterial, viral, fungal, protozoan or human antigen. More especially, the antigenic sequence may be from tetanus, hepatitis A, B or C virus, human rhinovirus such as type 2 or type 14, herpes simplex virus, poliovirus type 2 or 3, foot-and-mouth disease virus, influenza virus, coxsackie virus, HIV, HPV or *Chlamydia trachomatis*. Useful antigens include *E*. *coli* heat labile toxin B subunit (LT-B), *E*.*coli* K88 antigens, any protein from *B*. *pertussis* (e.g. P.69) and tetanus toxin fagment C. The antigen may be a cancer, malaria, measles, mycobacterium, diptheria, lieshmania, salmonella, treponema, mumps, shigella, neisseria, borrelia, rabies or cholera antigen.

A DNA construct comprising the promoter operably linked to DNA encoding the heterologous antigen may be made and transferred into the mycobacterium using conventional techniques. Transformants containing the DNA construct may be selected, for example be screening for a selectable marker on the construct. Mycobacteria containing the construct may be grown *in vitro* before being formulated for administration to the host for vaccination purposes.

### Cultures of the mycobacterium

The mycobacterium may be present in the form of a culture which also comprises a culture medium. Such a culture medium will comprise water, and generally also inorganic (such as sodium, potassium, calcium, magnesium, iron, selenium, carbonate, phosphate and/or sulphate ions). The culture medium will also comprise nutrients, typically in the form of an assimible carbon source, such as a carbohydrate source or amino acids. In one embodiment the culture medium also comprises one or more nutrients for which the mycobacterium is auxotrophic. Such as culture medium may thus comprise the amino acid normally (in the wild-type) synthesised or made available by the product of the mutated gene.

The culture generally comprises at least 100 mycobacterium cells, such as at least 10³, 10⁵, 10⁷ or 10⁹ mycobacterium cells.

The mycobacterium may be in substantially purified or isolated form (in vitro). Typically such a mycobacterium will be comprise at least 80%, such as at least 90, 95, 98 or 99% of the cells (e.g. of the bacterial cells) or dry weight of the mixture. In one embodiment the mycobacterium is present within the cells of the host which are being cultured in vitro. Such cells are typically cells which can be infected by the mycobacterium, such as macrophages or dendritic cells.

### Administration to a host

The mycobacterium can be administered to a host to prevent or treat a disease. The disease is typically tuberculosis caused by the pathogen *Mycobacterium tuberculosis* and/or other TB complex organisms, such as *M*. *bovis.* As discussed above in relation to mycobacteria which express heterologous antigens, the disease may be caused by another pathogen. Thus administration of an effective non-toxic dose of the mycobacterium will cause vaccination of the host against infection. Typically the mycobacterium is administered in the form of a vaccine formulation, for example in association with a pharmaceutically acceptable carrier or diluent.

The dosage employed will be dependent on various factors including the size and weight of the host and the type of vaccine formulated. However, a dosage comprising an administration of from 10² to 10¹¹, typically 10⁶ to 10⁸, mycobacteria per dose may be convenient for a 70kg adult human host. Any suitable route of administration may be used, for example oral (including nasal), parenteral, intravenous or subcutaneous.

### Examples

The following Examples serve to illustrate the invention.

### Example 1

### Materials and methods

### Mice

Female mice between 8-10 weeks of age were used in all experiments. Experimental animals were maintained in micro-isolator cages (Laboratory Care Precision, UK) until use, when they were transferred to negative pressure flexible film isolators following infection with *M. tuberculosis* in a Class I/III biohazard safety cabinet.

### Bacterial strains and media

Bacterial strains used in this study are outlined in Table I. *M*.*tuberculosis* H37Rv was grown in Middlebrook 7H9 broth (Difco) containing 0.05% Tween 80 and 10% OADC (Oleic acid albumin dextrose catalase) supplement (Becton Dickinson, UK) or Middlebrook 7H10 agar (Difco) plus 10% OADC supplement. Defined auxotrophic mutants of H37Rv were generated using homologous recombination. These had a disruption in the genes for biosynthesis for either methionine, proline or tryptophan synthesis and an insertion of the hygromycin resistance gene (Parish *et al* (1999) Microbiology 145, 3497-3503). Media and agar were supplemented with the appropriate individual amino acid proline or tryptophan at 50µg/ml for auxotroph growth. Auxotrophy or differentiation from wild type H37Rv *in vivo* was confirmed by plating liquid cultures onto 7H10/OADC plates with and without individual amino acids or hygromycin at 100ug/ml. In addition colony forming units were counted 4 weeks post autopsy and plating and checked at 6 weeks to control for any lag in growth on plates *ex vivo* for the mutants.

Freeze-dried live *M*. *bovis* BCG was obtained from the Staten Serum Institute, Denmark and reconstituted in normal saline prior to use.

**Table 1**

| Strain | Genotype | Phenotype | Reference |
|---|---|---|---|
| M.tb H37Rv | n/a | none | ATCC 25618 |
| M.tb H37Rv TAME1 | *metB::hyg* | none | Parish 1999 |
| M.tb H37Rv TAME2 | *proC::hyg* | proline auxotroph | Parish 1999 |
| M.tb H37Rv TAME3 | *trpD::hyg* | tryptophan auxotroph | Parish 1999 |
| *M*. *bovis* BCG Danish | | | |
| Strain 1331 | n/a | none | Staten Serum Institute |

### In vitro growth curves.

*M*. *tuberculosis* strains were grown in 10 ml of liquid medium until an OD₆₀₀ of between 0.5 and 0.8 were reached. These cultures were then used as an inoculum to give a theoretical OD₆₀₀ of 0.012. Twenty 50 ml conical tubes containing 10 ml each of liquid medium were inoculated for each strain and left standing without agitation of 37°C. The OD₆₀₀ was measured at regular intervals using a fresh tube for each reading.

### Infection of mice and tissue analysis

Viable stocks of H37Rv and auxotrophs were grown as described above, washed twice in phosphate buffered saline (PBS), resuspended and stored at -70°C. Mice were infected with 1x10⁶ viable mycobacteria in 200 µl normal saline via a lateral tail vein. Where appropriate, infected mice when moribund and terminally ill were killed by cervical dislocation in accordance with humane endpoint protocols under the Animals Scientific Procedures Act, 1986 (UK). Median survival times were calculated for each group and statistical analysis performed using the Log Rank tests of survival.

For tissue analysis, mice were killed by cervical dislocation and lungs, livers and spleens from individual animals, were collected aseptically and passed through a 100 micron pore size sieve (Falcon) in 7H9 medium containing 0.05% Tween 80. Colony forming units (CFU's) were determined from serial ten-fold dilutions in 7H9 on Middlebrook 7H10 agar plates supplemented with OADC, and where appropriate with amino acid. These were incubated for 3-4 weeks at 37°C prior to counting. For histological analysis, tissues were fixed in formol buffered saline, paraffin embedded and 1-3 µm sections cut using a Reichert-Jung 2030 microtome. Sections were stained with Haematoxylin and Eosin or Ziehl Neelsen and photographed using a Reichert-Jung Polyvar microscope.

### Analysis of protective efficacy of auxotrophic M. tuberculosis against challenge with H37Rv

DBA/2 mice were immunised intravenously (i.v.) with 1 x 10⁶ BCG or auxotrophic mutants of H37Rv. Six weeks post infection mice were challenged i.v. with 1 x 10⁶ CFU's of H37Rv. Mice were also taken prior to challenge infection to quantitate the residual tissue burden of the auxotrophs or BCG. After 4 and 8 weeks, mice were killed (n=6 per group at each time point) and tissue bacterial loads from lungs, livers and spleens were analysed by plating either on 7H10 plates or on selective 7H10 plates containing 5 µg/ml Triophen-2-carboxylic acid hydrazide, (Sigma), to differentiate H37Rv from BCG (Vestal and Kubica (1967) Scand. J. Resp. Dis. 48, 142-8). Alternatively tissues were plated on media containing 100ug/ml hygromycin and where appropriate 50ug/ml amino acid to distinguish between H37Rv and auxotrophic mutants in individual organs.

### In vitro infection of macrophages.

Bone marrow-derived macrophages were generated by culturing bone marrow cells harvested from the femurs of adult BALB/c mice in the presence of L cell conditioned medium for 8 days as described previously (Bancroft *et al*. (1994) Methods of Cell Biology Vol 45, Academic Press). Adherent cells were harvested and plated at a density of 1 x 10⁶/ml in 24 well plates (NUNC) in Dulbecco's modified Eagles medium (DMEM) supplemented with 10% foetal calf serum, 4500 mg glucose/litre, 100 mg sodium pyruvate/L-pyrodoxin. HCl, NaHCO₂ and 4 mM glutamine, in the absence of penicillin and streptomycin. Cells were infected with a multiplicity of infection (MOI) of one bacterium per cell (from bacterial stocks made as described above) for 4 hours before washing 6 times in warm tissue culture medium. The infection dose was assayed independently by plating the inoculum. At this time (taken as time =0) or at various intervals over a 14 day period, the number of viable mycobacteria was assessed on 7H10 plates, after lysis of the macrophage monolayer with 1 ml of sterile distilled water containing 0.1% Triton X-100 per well. The relevant amino acid was used to supplement plates where necessary.

### Statistical analysis

Statistical analysis was performed for bacterial CFU data using students t test and for survival curves using Kaplan Meier plots and Log Rank tests.

### Example 2

### Effects of auxotrophy on mycobacterial growth in culture

Our initial experiments compared growth curves for wild type H37Rv versus auxotrophic mutants of *M*. *tuberculosis* in liquid media to check whether strains had differing growth rates, which could influence an *in vivo* phenotype. Cultures were monitored in the presence or absence of the appropriate amino acid supplements, TAME1 is a prototrophic strain, despite the loss of the *metB* gene, and showed no significant difference to wild type growth rates (Figure 1A). Since this mutant has no phenotype, we have considered it as a control for the effects of genetic manipulation, particularly the presence of the hyg gene. In the absence of specific amino acid supplementation, TAME2 (*proC* mutant) and TAME3 (*trpD* mutant) did not grow (data not shown). TAME2 (Figure 1b) and TAME3 (Figure 1c) showed comparable growth rates (when grown in appropriately supplemented media) to wild-type H37Rv grown in media without amino acids. Interestingly, when L-proline or L-tryptophan was added to the medium, the wild type showed a distinct decrease in growth rate and in both cases the mutant grew faster. Nevertheless the result demonstrate that the mutants have similar intrinsic growth rates to the wild type strain and thus any *in vivo* differences seen are likely to be caused by other factors.

### Effect of auxotrophic mutation on the intracellular multiplication of M.tuberculosis.

To determine the effect of mutation in genes for amino acid biosynthesis on intracellular survival, murine bone marrow derived macrophages were infected at a multiplicity of infection of approximately 1:1 with either parental wild type H37Rv or mutant strains. At intervals over 14 days cells were lysed and viable bacteria counted. Wild type bacterial counts remained at around 10⁶ CFU/ml over the 12 days of assay (Table 2). Similarly the *metB* mutant remained stable between 1 - 3x10⁶ CFU/ml throughout the course of the experiment (Table 2). In contrast, a 25-fold reduction in viable mycobacteria recovered from the macrophages was seen for both the *proC* mutant (from 2.5 x 10⁵ to 1 x 10⁴ CFU/ml), and for the *trpD* mutant a 10-fold reduction in numbers was seen from 1.3 x 10⁵ to 1.3 x 10⁴ CFU/ml over the 12 days. Culture medium alone (DMEM, supplemented as in materials and methods) could not support the extracellular growth of mycobacteria. These results suggest that disruption of the *trpD* or *proC* genes alters the ability of *M*. *tuberculosis* to multiply in murine macrophages. The *metB* mutant controlled for the effects of genetic manipulation and demonstrated that the effect is likely to be due to auxotrophy in the two other candidates.

### Virulence of auxotrophic M. tuberculosis is SCID mice

A requirement of live attenuated vaccines is that they should be safe even when used in immunocompromised hosts. To test for bacterial multiplication in the absence of specific immunity, SCID mice, lacking both T and B cells and previously shown to be highly susceptible to *M*.*tuberculosis* infection (North and Izzo. (1993) Am. J. Pathol. 142, 1959) were inoculated with wild type H37Rv or auxotrophic mutants derived in the same strain. Table 3 shows that all mice infected with H37Rv became moribund and died between days 28 and 29. The mice infected with *metB* showed a slight increase in length of survival (a median of 42 days). In strong contrast, mice infected with the *proC* mutant survived significantly longer (p<0.001) with a median time to death of 130 days. Only one death was seen in the group infected with the *trpD* mutant (at 241 days) during the course of the experiment which was terminated at 301 days. At this time the *trpD* infected mice appeared clinically healthy although an autopsy showed histological evidence of granuloma formation in the liver associated with the presence of mycobacteria. Importantly, no lesions or mycobacteria were observed in the lung tissue of these mice on histological analysis.

Thus a significant reduction in virulence of auxotrophs for proline and tryptophan was seen compared to H37Rv wild type in immunodeficient mice.

### Growth kinetics of H37Rv versus auxotrophic M. tuberculosis in tissues of SCID mice.

To monitor the effects of auxotrophy on the replication of *M*. *tuberculosis in vivo,* SCID mice were infected with either H37Rv, *metB*, *proC* or *trpD* and bacterial burdens assays in the liver, lungs and spleens. Bacterial numbers recovered from all organs of the mice infected with wild type H37Rv rose rapidly to Log₁₀7.5 in the liver, Log₁₀9 in the spleen and Log₁₀9.2 (an irreversible level of bacteraemia) in the lung by day 22 (Table 4). The three remaining mice in this experimental group died by day 29 confirming the results in Table 3. Bacterial growth was significantly delayed (p<0.01) in the group infected with *metB*, but followed a similar kinetic or *in* *vivo* growth rate as wild type in all three organs, and the three remaining mice in this experimental group were dead by day 45, confirming previous survival data (Table 3).

In contrast, the rise in bacterial burden was significantly delayed in mice infected with the *proC* mutant consistent with their increased survival times. Thus at day 20, the *proC* mutant was significantly attenuated *in vivo* both in the liver and spleen (p<0.0001) and also in the lungs (p<0.01) compared to wild type H37Rv. Mice infected with *proC* demonstrated a degree of control over bacterial replication in the liver and spleen but importantly they demonstrated a gradual loss of control in the lung after day 20 (Table 4).

Mice infected with *trpD* had consistently lower bacterial burdens compared to either H37Rv or *proC* at all time points examined, consistent with their prolonged survival. Although bacterial numbers in the liver of SCID mice infected with *trpD* were maintained around Log₁₀5, in both the spleen and lung there was rapid decrease in organ load post infection to around or below the limits of detection (100 bacteria per organ). This was maintained until day 90, the termination of the experiment.

In summary, *proC* was attenuated *in vivo* but capable of replication, particularly in the lungs, after day 35. The *trpD* mutant was controlled or stabilised in all three organs, remaining around or below the level of detection in both lung and spleen.

### Cellular infiltration and granuloma formation in the lungs and liver of SCID mice infected with wild type and mutants

To test whether the difference in progression of disease and bacterial burdens between H37Rv and auxotrophic mutants was accompanied by visible changes in the tissues, the histological response was examined over the course of infection. It has previously shown that SCID mice are capable of granuloma formation in response to mycobacterial infection. The livers of mice infected with wild type H37Rv showed granuloma formation focussed on areas of mycobacterial growth, with both the size and number of lesions and the evidence of mycobacteria reflecting the rapid progression to death by day 29. In the *metB* mice there was a delay in granuloma formation and size, in keeping with the slight delay in disease progression to death. No granulomas were seen in the liver in either the *proC* or *trpD* groups at this time. By day 35 granulomas had become more extensive in the *metB* group, small and rare in the *proC* group and none were seen in the *trpD* group. By day 90 in the *proC* mice there were a few very small granulomas but no visible mycobacteria in the liver. Only by day 90 were a very few granulomas containing greater than ten inflammatory cells with visible mycobacteria seen in SCID mice infected with *trpD.*

Lungs of SCID mice infected with H37Rv, *metB*, *proC* or *trpD* on day 22 were stained with ZN for acid fast bacteria and counterstained to visualise inflammatory responses. In the lungs the histological response to H37Rv was characterised by massive cellular infiltration with thickening of the airway epithelia and 20-30% of the tissue occupied by well demarcated granulomas by day 22. This cellular infiltration was overwhelming by day 29, the time of death. This response was delayed in the *metB* mutant infections and the lesions were smaller and fewer in number at day 22. Whilst the lung granulomas were less extensive in the *metB* mice, they were also accompanied by a thickening of the lung epithelium and the presence of visible mycobacteria in the lesions, again reflective of large numbers of mycobacteria in these organs. In stark contrast to the wild type or *metB* infected mice no visible mycobacteria or lesions were seen in either the *proC* (albeit there was evidence of thickening of the lung epithelium) or *trpD* at 22 days or 35 days. In the lungs of the *proC* infected mice at day 90 a few small discrete granulomas were seen with visible mycobacteria and in the *trpD* infected mice no bacteria or granulomas were seen at day 90.

### Survival of immunocompetent mice infected with auxotrophic mutants.

To determine whether the loss of genes involved in amino acid biosynthesis affected the growth rate of *M*. *tuberculosis* in the immunocompetent host, DBA/2 mice were infected with either wild type H37Rv or auxotrophs *metB*, *proC* or *trpD*. Mice infected with wild type died between 78 and 100 days post infection (median 83.5 days). In contrast 2/5 mice infected with *metB* survived for over 350 days (Table 5). All *proC* or *trpD* infected mice survived for the duration of the experiment, which was terminated at 350 days. In a parallel experiment conducted in susceptible 129SvEv mice, H37Rv infected mice survived for a median of 240 days and there were no deaths in the other groups. This demonstrated that this strain was more resistant than DBA/2. Thus although H37Rv was virulent in DBA/2 mice, *metB* was somewhat attenuated and mice infected with *proC* and *trpD* appeared to be unaffected by the infection suggesting these mutants were significantly attenuated *in vivo.*

### Growth kinetics of H37Rv versus auxotrophic M. tuberculosis in tissues of immunocompetent mice.

Having determined that immunocompetent mice infected with auxotrophic mutants survived as long as uninfected controls, we monitored mycobacterial growth in their tissues. The course of infection in both the H37Rv and *metB* infected mice was remarkably similar in all organs and statistical analysis revealed no differences in tissue burdens at any time. Thus bacteria were stable or controlled in the liver and spleen and notably increased in the lungs from Log₁₀5 to log₁₀7.4 for H37Rv and Log₁₀6.9 for *metB* over 90 days (Table 6). In contrast to the control of high levels of infection seen in wild type or *metB* infected mice the numbers recovered from the livers of *proC* infected mice were significantly lower, by two logs, at day 14 (P<<0.0001). Over the course of the experiments these rose by approximately ten fold to equal the number recovered from mice infections with *M*. *tuberculosis,* the numbers of bacteria recovered from *trpD* infected mice fell ten fold over the 90 days, further evidence of attenuation.

In the spleens of mice infected with wild type H37Rv or *metB* bacterial numbers were stable. After an initially low recovery of *proC* at day 14 numbers rose to the levels recovered from wild type infected mice by day 30. In the spleen as in the lung the numbers of *trpD* dropped over the 60 days by three logs.

The lung CFU's reflect the spleen data. In marked contrast to the course of infection in DBA mice infected with H37Rv (or *metB*) at day 14 no bacteria were recovered from the lungs of *proC* infected mice. However by day 45 some of the mice had recoverable bacteria in the lungs and significant numbers were present in all mice in the group. In contrast, in mice infected with *trpD* significant numbers were recovered at the first time point (14 days) but after this time numbers remained below the levels of detection suggesting clearance occurred in this organ. Thus loss of the ability to synthesise proline or tryptophan rendered these auxotrophs less virulent than H37Rv and *trpD* was more severely attenuated compared to *proC*.

### Granuloma formation in the lungs and livers of immunocompetent mice infected with wild type or auxotrophic mutants.

Granuloma formation is a key component of the adaptive response to mycobacterial infection and also dependent on tissue burden and virulence of the mycobacterial infection. Therefore we addressed the question of whether the persistence of mycobacteria affected the growth and development of granulomas in the tissues. Granulomas in the livers of mice infected with H37Rv at day 30 consisted of between 10-20 cells and occasional visible mycobacteria were seen-these persisted until day 90. Mice infected with *metB* had more numerous granulomas at day 30, although at this time these were less focused with few or no visible mycobacteria. These became more focused throughout the course of infection and by 90 days most granulomas consisted of about 10 cells still with no visible mycobacteria. In mice infected with *proC* only a few very small (<5cells) granulomas were seen at day 30. By day 45 there were still very few granulomas with about 10-15 cells, and by day 90 there was little evidence of any pathology in the liver. *TrpD* infected mice only developed a small number of granulomas by day 90, none were seen at days 30 and 60.

Analysis of the lung tissue showed that by 30 days of infection the mice infected with wild type *M*. *tuberculosis* had signs of thickening of the lung airways associated with pneumonia. By day 58 there were visible granulomas which contained mycobacteria. By day 90 the granulomatous areas spread over 20% of the tissue with clefts of lymphocytes and evidence of a neutrophil influx. This was classified as stage 3 (Rhoades, Frank and Orme, (1997) Tuber. Lung Dis. 78(1), 57-66) and mycobacteria could clearly be seen with necrosis in some areas. Histological changes in the lungs of *metB* infected mice followed a similar but delayed course. By day 30 there was some thickening evident which progressed to early granulomas by day 45. There was an increase in size and visible mycobacteria by day 58 and there was extensive granulomatous involvement by day 90. By day 90 these differences between mutants and H37Rv were very clear. In contrast no obvious inflammatory responses or mycobacteria were visible in the lungs of mice infected with *proC* or *trpD* at any time during the course of the experiment.

### Protective efficacy of auxotrophic mutants against challenge with virulent M. tuberculosis.

Having determined that the auxotrophs *proC* and *trpD* were significantly attenuated we tested whether they had protective efficacy, compared with BCG, against challenge with H37Rv. DBA/2 mice were infected with either *proC*, *trpD* or 10⁶ BCG i.e. and challenged with wild type H37Rv six weeks later. Mice were also taken at time of challenge to estimate the residual tissue burdens of BCG or auxotroph (Table 7). After 4 weeks quantitation of CFU's in the liver and spleen showed that both auxotrophs conferred protection apparently equal to that seen with BCG (Table 7). The number of wild type versus auxotroph *M*.*tuberculosis* at each time point post challenge were quantitated using differential plating methods as described in materials and methods - one month: *proC -* lung log₁₀ 4.4; liver log₁₀ 5.2; spleen log₁₀ 4.6; *trpD*-lung log₁₀ 1.5; liver log₁₀ 4.2; spleen log₁₀ O. At 2 months the residual tissue burdens of the innocula were *proC -* lung log₁₀ 4.2; liver log₁₀ 5.2; spleen log₁₀ 4.8; *trpD -* lung log₁₀ O; liver log₁₀ 3.1; spleen log₁₀ 1.7. These strongly reflected the data shown in Table 6 for survival of auxotrophs in otherwise unchallenged mice.

In the liver, all three vaccinations significantly reduced the numbers recovered after challenge compared to infected controls. This represented an 85% reduction in H37Rv recovered from mice vaccinated with either BCG (p<0.0008) or *trpD* (p<0.0001) and 80% reduction for *proC* vaccination (p<0.0024). In the spleen the numbers of H37Rv recovered were also significantly reduced in vaccinated mice, 67% for BCG vaccination (p<0.0013); 60% for *trpD* (p<0.02) and 70% for *proC* vaccination (p<0.0039). At this time point no significant reduction in burden was seen in the lungs of mice previously infected with either BCG or auxotrophic *M.* *tuberculosis.*

However after two months highly significant levels of protection were found in the lung for both mutants in excess of the 67% for BCG vaccination (p<0.01). A 73% reduction in tissue burden was seen for *proC* (p<0.009) and as high as 86% reduction was seen in mice infected with *trpD* prior to challenge. Evidence of protection was seen in the liver at this time but not in the spleen.

Of the three auxotrophs described here unexpectedly the mutant containing a disrupted gene for *metB* was a prototrophic strain having no requirement for exogenous methionine in axenic culture. One possible explanation is the presence of another gene or enzyme which can complement or compensate for the loss of *metB* activity. With appropriate amino acid supplementation *trpD* and *proC* had similar intrinsic growth rates to H37Rv and thus differences seen in the ensuing experiments were likely to be caused by bacterial location within the cell or external availability of exogenous amino acid. As such methionine and tryptophan, but not proline which is synthesised by a special pathway, represent essential amino acids not yet synthesised in mammalian systems. Since the *metB* mutant was not auxotrophic, and unlikely to have a phenotype this was considered as a control for the effects of genetic manipulation.

Interestingly, despite this *metB* showed the same kinetics of growth in macrophages as H37Rv. Both *proC* and *trpD* were attenuated however in this model of *in vitro* infection. It has been shown that *leuD* auxotrophs of both *M*. *tuberculosis* were restricted in their growth in macrophages, maybe due to sequestration after phagocytosis in an intracellular compartment from which they cannot obtain leucine. In this case leucine was provided as a supplementation to the media employed for macrophage culture. In our case external availability of amino acid in the media was considered of potential relevance - it contained 30µg/ml methionine, 16µg/ml tryptophan but no proline. The relevance of this to the *in vivo* situation is not known, since we do not know what concentrations of amino acids are available in the internal environment of the macrophage, particularly within the phagolysosome.

Attenuated strains are generally more potent that non-living vaccines in stimulating cell mediated immune responses effective against intracellular pathogens, indeed BCG was originally employed as a vaccine not only on the basis of its safety but because of its relatedness to *M*. *tuberculosis.* Nowadays a requirement of an attenuated strain of *M*. *tuberculosis* is that it would not survive long enough to cause pathology or disease even in immunodeficient individuals, who are particularly at risk from *M. tuberculosis.* Thus to test these auxotrophic mutants for their potential for vaccine candidates we selected only immunocompetent mice but also mice with severe combined immunodeficiency disease (SCID). H37Rv is extremely virulent in these mice and *metB* proved to be also albeit with a slight delay in death. Both *proC* and *trpD* were significantly attenuated in SCID mice with *trpD* being very severely attenuated, survival times exceeding 300 days for the majority of mice. These findings are similar to *leuD* which has been shown not only unable to replicate in macrophages but also to cause no deaths in SCID mice. In addition these prolonged survival times for SCID mice infected with *trpD* compare very favourably with those described for the course of infection with BCG in SCID mice which generally succumb by 8-10 weeks. From this it could be concluded that *trpD* may be safe to use in immunodeficient individuals.

The *proC* mutant showed evidence of retardation in the lung but loss of control in this organ contributed significantly to progression to death in mice infected with H37Rv or *metB*. There was, in contrast, evidence of long term control in the liver for both mutants. Importantly, *trpD* was controlled or stabilised in all three organs significantly remaining around or below the level of detection in the lung and spleen.

We and others have previously shown that SCID mice even in the absence of T cells are able to form granulomas and that this operates as a key part of the early response to infection. Extraordinarily, even in SCID mice there were minimal histological changes up until the termination of the experiment at day 90, in the lung tissue of mice infected with *proC* and none in mice infected with *trpD*. Thus virulence in the SCID mouse is associated with clear changes, development of graanulomas and cellular infiltration, and attenuation reduces pathology in the lung to a minimum.

We then tested our mutants for survival and growth in immunocompetent mice. DBA/2 mice are known to be more susceptible to infection with *M*. *tuberculosis* than the major histocompatibility complex compatible BALB/c mice. They tend to be more susceptible in the lung developing more lung pathology but express equal ability to express immunity in the liver and spleen. In our hands it was found that infection with H37Rv led to death in 50% DBA/2's within 100 days similar to previous reports. This allowed us to demonstrate the prolonged survival, over 300 days, or mice infected with either *proC* or *trpD*. When immunocompetent mice were infected with the mutants even mice infected with *metB* survived longer than H37Rv suggesting some sort of attenuation. The kinetics of *in vivo* bacterial growth for *metB*, however, closely resembled those of H37Rv. This reflected previous date for an auxotroph of BCG mutated for *met 2,* a homologue of the spb gene in cyanobacteria which grew *in vivo* in BALB/c mice almost to the level of BCG, and conferred protection equal to BCG itself.

Mice infected with either *proC* or *trpD* demonstrated significant long term survival. The data is for growth *in vivo* was most striking in the lung in that whereas *metB* remained at similar numbers to H37Rv over the 90 days, *proC* was originally detected at very low levels, maintained for 30-40 days and increased over the course of infection. In contrast *trpD* was controlled after an early time point and then throughout the 90 days. This was reflected in the histological analysis at day 90 where no overt changes were seen in the lungs of mice infected with either *proC* or *trpD* compared with H37Rv infection where granulomas were clearly visible. These granulomas were discretely spread over the lung area and seemed to coalesce in later stages of infection. It is thought that in the mouse, as in humans, tuberculosis is a chronic disease of the lungs, the only organ in which the organism causes excessive pathology. This is true in the mouse model regardless of whether the infection is initiated via the respiratory or intravenous route. Of interest is the fact that the mutants had very different kinetics not only between each other but also in different organs and the data strongly supports the theory that the lung is the key organ for demonstrating virulence of isolates.

The choice of DBA/2 was maintained in the testing of these mutants as vaccine candidates. Vaccination clearly provides this strain with a capacity to reduce the level of infection in their lungs. However vaccinated DBA/2 mice remain more susceptible than BALB/c mice developing more lung pathology and dying much earlier. These mice retain a similarly increased an equal ability to express immunity in the liver and spleen indicating an equal systemic level of vaccine induced immunity. We were able to show significant protection in all organs, with liver burdens significantly reduced at both 4 and 8 weeks after challenge. The numbers of H37Rv harvested from the spleen were only significantly reduced one month after challenge but importantly in the lung they were significantly reduced 2 months after challenge with greater levels of protection in this organ than seen with BCG. This compares very favourably with other auxotrophs of *M*. *tuberculosis, leuD,* which confer less protection than BCG, and *purC* which produces equal protection in the lung but not in the spleen.

Uniquely we also made observations as to the residual tissue burden of mutants, both at the time of challenge and one and two months after, these strongly reflecting the kinetics that we had seen in mice without the overlay of challenge infection. Of real interest was that the latter point the *trpD* derived from the immunisation was reduced below detectable levels in the lung an organ in which the highest degree of protection against H37Rv was obtained. To induce optimal protection attenuated strains of *M*. *tuberculosis* need to both persist for some time, to home to an appropriate organ to engender immunity and be sufficiently metabolically active to synthesise relevant antigens for an extended period of time. The acquired phase of immunity is driven in response to mycobacterial infection by T cells and in terms of *in vivo* infection in mice on onset of control in immunocompetent mice correlates with effective granuloma formation observed in the liver at about 4 to 6 weeks.

Our data for auxotrophs of *M*. *tuberculosis* contrasts with that of Jackson *et al*. (1999) Infection and Immunity 67(6), 2867-73, for the mutant of *M*.*tuberculosis purC* which was eliminated from the liver. In our study *proC* rose one log to equal the numbers found in the H37Rv infected mice by the termination of the experiment although the *trpD* did fall by one log it never fell below detectable limits in this organ. Thus both these mutants persisted in the liver. This persistence may be one possible mechanism for the higher levels of protection, equivalent to BCG that we were able to see in later experiments. However, importantly we did get *trpD* clearance from the lung and the spleen even on challenge with H37Rv.

The study attempted to identify dissemination, persistence, pathology and protection responses to infection with auxotrophs of *M*. *tuberculosis.* These mutants engendered statistically equivalent or better reduction in bacterial loads in the lung on challenge with H37Rv compared with levels attained with BCG. What has been shown in this study is that auxotrophy is capable of severely attenuating *M tuberculosis* and providing protection against challenge infection, with one mutant, *trpD* appearing less virulent than BCG in the lung and in addition strongly protective.

**Table 2:**

| **Effect of auxotrophic mutation on the intracellular multiplication of *M. tuberculosis*** | | | | |
|---|---|---|---|---|
| Bacterial counts from bone marrow derived macrophages infected with parental strain WT; *metB; proC or trpD.* Results are the means of three different measurements per time point taken over 11 days. | | | | |

| **Day post infection** | **WT** | ***metB*** | ***proC*** | ***trpD*** |
|---|---|---|---|---|
| **0** | 150 x 10⁴ | 335 x 10⁴ | 25 x 10⁴ | 13 x 10⁴ |
| **1** | 68 x 10⁴ | 335 x 10⁴ | 53 x 10⁴ | 7 x 10⁴ |
| **2** | 68 x 10⁴ | 305 x 10⁴ | 25 x 10⁴ | 3 x 10⁴ |
| **5** | 100 x 10⁴ | 305 x 10⁴ | 18 x 10⁴ | 5 x 10⁴ |
| **8** | 48 x 10⁴ | 83 x 10⁴ | 3 x 10⁴ | 2 x 10⁴ |
| **11** | 66 x 10⁴ | 121 x 10⁴ | 1 x 10⁴ | 1x10⁴ |

**Table 3:**

| **Virulence of auxotrophic *M*. *tuberculosis* in SCID mice** | | | | |
|---|---|---|---|---|
| Mice were infected intravenously with 1x10⁶ of H37Rv; or mutants *metB*, *proC*, *trpD* and survival monitored over 11 months. Each group contained 6 mice and results are representative of two separate experiments. Counts for the innocula were; WT 3x10⁶; *metB* 1x10^{6;} *proC* 1 x 10⁶;*trpD* 3x10⁶. Numbers represent the percentage of mice in each group surviving at intervals post infection. | | | | |

| **Day post infection** | **H37Rv** | ***metB*** | ***proC*** | ***trpD*** |
|---|---|---|---|---|
| **1** | 100 | 100 | 100 | 100 |
| **28** | 83 | 100 | 100 | 100 |
| **29** | **0** | 100 | 100 | 100 |
| **39** | | 83 | 100 | 100 |
| **40** | | 67 | 100 | 100 |
| **42** | | 33 | 100 | 100 |
| **47** | | **0** | 100 | 100 |
| **120** | | | 67 | 100 |
| **127** | | | 50 | 100 |
| **132** | | | 33 | 100 |
| **143** | | | 17 | 100 |
| **156** | | | **0** | 100 |
| **241** | | | | 83 |
| **301** | | | | **83** |

**Table 4:**

| **Effect of mutation on the survival and multiplication in the lung, liver and spleen of SCID mice** | | | | |
|---|---|---|---|---|
| Mice were infected with 1x10⁶ wild type H37Rv; or auxotrophs of *M*. *tuberculosis ; metB*, *proC*, *trpD*. CFUs were assayed at various intervals on 7H10 with or without appropriate amino acid supplementation. Innocula of all *in vivo* infections (t=0) were similarly checked to ensure parity of infection between groups of mice, the results were Log₁₀ 6.2 for H37Rv; *metB* Log₁₀5.8; *proC* Log₁₀6.1 and *trpD* Log₁₀ 6. The results represent means and standard errors for three mice per group and significance was measured using student's t test. Results are representative of two separate experiments. | | | | |

| **4.1 Lung CFUs** | | | | |
|---|---|---|---|---|
| **Day post infection** | **H37Rv** | ***metB*** | ***proC*** | ***trpD*** |
| **10** | 5.87+0.42 | 3.68+0.7 | 3.24+0.76 | 0.77+0.94 |
| **22** | 9.18+0.02 | 6.41+0.55 | 2.6+1.62 | 0.67+0.82 |
| **35** | | 8.43+0.29 | 4.47+0.2 | 1.67+0.41 |
| **70** | | | 6.65+0.12 | 2.17+1.34 |
| **90** | | | 7.59+0.2 | 2.7+0 |

| **4.2 Liver CFUs** | | | | |
|---|---|---|---|---|
| **Day post infection** | **H37Rv** | ***metB*** | ***proC*** | ***trpD*** |
| **10** | 6.08+0.09 | 4.1+1.5 | 5.16+0.29 | 4.19+1.05 |
| **22** | 7.51+0.34 | 7.05+0.24 | 5.61+0.25 | 5.2+0.15 |
| **35** | | 8.68+0.19 | 6.17+0.22 | 4.85+0.09 |
| **70** | | | 6.75+0.96 | 5.6+0.19 |
| **90** | | | 7.32+0.01 | 5.77+0.32 |

| **4.3 Spleen CFUs** | | | | |
|---|---|---|---|---|
| **Day post infection** | **H37Rv** | ***metB*** | ***proC*** | ***trpD*** |
| **10** | 7.09+0.22 | 5.17+1.12 | 5.19+0.96 | 4.84+0.75 |
| **22** | 8.99+0.45 | 8.11+0.05 | 5.36+0.47 | 1.03+1.27 |
| **35** | | 9.21+0.18 | 5.63+0.12 | 2.47+0.29 |
| **70** | | | 6.73+0.15 | 3.32+0.36 |
| **90** | | | 7.25+0.01 | 3.21+055 |

**Table 5:**

| **Survival of immunocompetent mice infected with auxotrophs** | | | | |
|---|---|---|---|---|
| DBA mice were infected intravenously with 1x10⁶ of H37Rv or mutants of H37Rv, *metB*, *proC*, *trpD* and survival monitored over eleven months. The results are representative of two separate experiments with 5/6 mice per group. | | | | |

| **Day post infection** | **H37Rv** | ***metB*** | ***proC*** | ***trpD*** |
|---|---|---|---|---|
| **1** | 100 | 100 | 100 | 100 |
| **79** | 83 | 100 | 100 | 100 |
| **80** | 67 | 100 | 100 | 100 |
| **82** | 50 | 100 | 100 | 100 |
| **85** | 33 | 100 | 100 | 100 |
| **94** | 17 | 100 | 100 | 100 |
| **100** | **0** | 100 | 100 | 100 |
| **145** | | 80 | 100 | 100 |
| **196** | | 60 | 100 | 100 |
| **222** | | 40 | 100 | 100 |
| **350** | | 40 | 100 | 100 |

**Table 6:**

| **Effect of mutation on the survival and multiplication in the lung, liver and spleen of DBA mice** | | | | |
|---|---|---|---|---|
| Mice were infected with 1x10⁶ wild type or auxotrophs of *M*.*tuberculosis*. Innocula of all *in vivo* infections (t=0) were checked to ensure parity of infection between groups of mice, the results were Log₁₀ 6.69 for H37Rv; *metB* 6.54; *proC* Log₁₀6.48 and *trpD* Log ₁₀ 6.6. CFUs were assayed at various intervals on 7H10 with or without appropriate amino acid supplementation. The results represent means and standard errors for three mice per group and significance was measured using students t test. The results are representative of two separate experiments. | | | | |

| **7.1 Lung CFUs** | | | | |
|---|---|---|---|---|
| **Day post infection** | **H37Rv** | ***metB*** | ***proC*** | ***trpD*** |
| **14** | 5.32+0.06 | 5.09+0.02 | 0+0 | 3.58+0.5 |
| **30** | 5.98+0.01 | 5.91+0.02 | 2.1+1.05 | 0+0 |
| **45** | 6.21+0.21 | 6.16+0.06 | 2.97+1.49 | 0+0 |
| **58** | 6.48+0.07 | 6.59+0.22 | 4.84+0.06 | 0+0 |
| **90** | 7.37+0.22 | 6.90+0.16 | 5.10+0.11 | 2+0 |

| **7.2 Liver CFUs** | | | | |
|---|---|---|---|---|
| **Day post infection** | **H37Rv** | ***metB*** | ***proC*** | ***trpD*** |
| **14** | 6.31+0.21 | 6.58+0,16 | 5.01+0.61 | 5.72+0.05 |
| **30** | 5.93+0.06 | 6.41+0.26 | 5.64+0.02 | 5.08+0.06 |
| **45** | 5.66+0.07 | 5.73+0.1 | 5.45+0,1 | 5.19+0.06 |
| **58** | 5.41+0.02 | 5.57+0.11 | 5.67+0.06 | 4.48+0.08 |
| **90** | 5.20+0.06 | 5.36+0.16 | 5.58+0.11 | 4.70+0.17 |

| **7.3 Spleen CFUs** | | | | |
|---|---|---|---|---|
| **Day post infection** | **H37Rv** | ***metB*** | ***proC*** | ***trpD*** |
| **14** | 5.71+0.25 | 5.76+0.08 | 1.60+1.6 | 4.48+0.09 |
| **30** | 5.25+0.04 | 5.59+0.12 | 5.29+0.08 | 3.82+0.12 |
| **45** | 5.27+0.09 | 5.58+0.06 | 5.33+0.12 | 3.06+0.11 |
| **58** | 5.52+0.12 | 5.64+0.08 | 5.72+0.04 | 3.36+0.06 |
| **90** | 5.64+0.2 | 5.54+0.09 | 5.77+0.08 | 3.15+0.02 |

**Table 7:**

| **Protective efficacy of auxotrophs of H37Rv against challenge with virulent H37Rv *M.tuberculosis* in DBA mice** | | | | | |
|---|---|---|---|---|---|
| Mice were inoculated i.v. with 1x10⁶ *M.tuberculosis* auxotrophs or BCG or given saline alone as controls, and challenged i.v. at 6 weeks with 1x10⁶ H37Rv. mice were in groups of 6 per time point per treatment. CFUs from lungs, livers and spleens on immunised and M *tuberculosis* challenged mice. At the time of challenge the residual burden of innocula was determined:- BCG (liver Log₁₀ 3.057; lung Log₁₀ 0.767; spleen3.24); *proC* (Liver Log₁₀ 5.84; Lung Log₁₀ 4.44; Spleen Log₁₀ 5.64) and *trpD* ( Liver Log₁₀ 4.52; Lung Log₁₀ 0; Spleen Log₁₀ 2.89. Animals were killed at 4 or 8 weeks post challenge and homogenates of lungs, liver and spleens were serially diluted on differential plates, to distinguish between innoculum and challenge. Results represent mean +/- standard error for six mice per group. Statistical significance was measured using students t test. Data divided by 1x10⁴ | | | | | |

| | **Month post challenge** | **Saline** | BCG | ***proC*** | ***trpD*** |
|---|---|---|---|---|---|
| **LUNG** | **1** | 66.33+11 02 | 43.8+11.56 | 117.3+8.38 | 44.5+6.49 |
| | **2** | 2340+535 | 771.7+48.12 | 631.7+57,18 | 328.3+54.7 |
| **LIVER** | **1** | 46.67+6.49 | 6.94+0.7 | 9.53+1.58 | 7.04+1,67 |
| | **2** | 23.77+9.2 | 5.02+0.57 | 8.21+1.15 | 5.44+1,13 |
| **SPLEEN** | **1** | 7.967+1.19 | 2.58+0.46 | 2.54+0.84 | 3.18+1.26 |
| | **2** | 15.97+4.46 | 16.65 + 3.21 | 19.98+6.54 | 1.41+2.6 |

## Claims

1. Use of a mycobacterium attenuated by a mutation in a gene that expresses a product which promotes synthesis of proline or tryptophan in the manufacture of a medicament for vaccination against tuberculosis.

2. Use according to claim 1 wherein the mycobacterium is *Mycobacterium tuberculosis* or *Mycobacterium bovis*.

3. Use according to claim 1 or 2 in which the mutation is in the *proC* or *trpD* gene.

4. Use according to any one of the preceding claims wherein the mycobacterium is further attenuated by a mutation in a second gene.

5. Use according to claim 4 wherein the second gene is selected from the group consisting of *pirC*, *erp*, *pps*, *fadD28*, *mmpL7*, *hspR*, *fadD26*, *pks6*, *Rv2452c, Rv1395*, *lipF*, *drrrC*, *mmpL2*, *Rv0204*, *modA*, *mmpL4*, *Rv3081c*, *cmaA*, *narG* and *fbpA*.

6. Use according to any one of the preceding claims wherein the mutation in the gene defined in claim 1 and/or the mutation in the second gene is a non-reverting mutation.

7. Use according to any one of the preceding claims wherein the mutation in the gene defined in claim 1 and/or the mutation in the second gene is a defined mutation.

8. Use according to any one of the preceding claims wherein the mycobacterium does not contain any antibiotic resistance genes.

9. Use according to any one of the preceding claims wherein the mycobacterium is genetically engineered to express an antigen from another organism.

10. A mycobacterium as defined in any one of the preceding claims.

11. A mycobacterium as defined in any one of the preceding claims for use in a method of treatment of the human or animal body by therapy.

12. A mycobacterium according to claim 11 for vaccination against tuberculosis.

13. A culture comprising a mycobacterium as defined in any one of claims 1 to 9 and a culture medium which comprises the amino acid the synthesis of which is promoted by the said gene product.

14. A method of making a mycobacterium as defined in claim 1, comprising introducing a mutation as defined in claim 1 into the genome of a mycobacterium.

15. A method of replicating a mycobacterium as defined in claim 1, comprising culturing the mycobacterium in a culture medium which comprises the amino acid the synthesis of which is promoted by the said gene product.


SEQUENCE LISTING

<110> GLAXO GROUP LIMITED

<120> VACCINE

<130> N79741B GCW

<140>
<141>

<150> GB 0014845.2
<151> 2000-06-16

<160> 4

<170> PatentIn Ver. 2.1

<210> 1
<211> 1113
<212> DNA
<213> Mycobacterium tuberculosis

<400> 1
gtggcgctgt cagctgaggg ttcttcgggg ggatctcgcg ggggctcgcc gaaagcagaa 60
gccgcatcgg tgccgtcctg gccgcagatt ttggggcggt tgaccgacaa ccgcgatctg 120
gcgcggggcc aggctgcctg ggccatggac cagatcatga ccggcaatgc gcggccggcg 180
cagatcgccg ccttcgcggt ggcgatgacg atgaaggctc cgaccgccga cgaagtcggt 240
gagctcgctg gcgtcatgct cagccacgcg catccgttgc ccgccgatac ggtccccgac 300
gacgccgtcg acgtcgtcgg caccggtggc gacggagtca acacggtgaa cctatccacc 360
atggcggcga ttgtggtggc ggctgcgggt gtgccggtgg tcaagcacgg caaccgagcg 420
gcgtcctcgt tgtccggtgg cgccgacacg ctggaggcgc tgggggtgcg catcgacctg 480
ggacccgacc tggtcgcgcg cagcctcgcg gaggttggga tcgggttctg cttcgcgccg 540
cggttccatc cctcctaccg gcacgcggcc gcggtacgcc gcgagatcgg tgtgcccacc 600
gtgttcaatc ttctcgggcc gctgaccaat ccggcccggc cccgggctgg actgatcgga 660
tgcgcgttcg ccgacctcgc cgaagtgatg gccggggtgt ttgctgcgcg ccggtccagt 720
gtgctggtgg tacacggcga cgatgggctg gacgagttga ccaccaccac cacgagcacg 780
atctggcgcg ttgcggcggg cagcgtggat aagttgacgt ttgatcccgc cggattcggg 840
tttgcgcgcg cccagcttga ccagctggct ggaggcgatg cgcaagccaa cgcggccgcg 900
gtgcgcgcgg tgttgggcgg tgccaggggc ccggtccggg acgctgtggt gctcaacgcc 960
gccggtgcga tcgtcgccca cgccggatta tccagccgtg ctgaatggct gccggcctgg 1020
gaggaggggt tgcggcgggc cagtgcggca atcgacaccg gtgcggccga acagttgctc 1080
gcgcgatggg tgcggttcgg tcggcagatc tga 1113

<210> 2
<211> 2209
<212> DNA
<213> Mycobacterium tuberculosis

<220>
<221> misc_feature
<222> (434)...(439)
<223> unknown

<220>
<221> misc_feature
<222> (452)...(704)
<223> unknown

<400> 2
atgcttttcg gcatggcaag gatcgcgatt atcggcggcg gcagcatcgg tgaggcattg 60
ctgtcgggtc tgctgcgggc gggccggcag gtcaaagacc tggtagtggc cgagcggatg 120
cccgatcgcg ccaactacct ggcgcagacc tattcggtgt tggtgacgtc ggcggccgac 180
gcggtggaga acgcgacgtt cgtcgtcgtc gcggtcaaac cagccgacgt cgagccggtg 240
atcgcggatc tggcgaacgc gactgcggcg gccgaaaacg acagtgctga gcaggtgttc 300
gtcaccgtgg tagcgggcat cacgatcgcg tatttcgaat ccaagctacc ggctgggacg 360
ccagtggtgc gtgcgatgcc gaacgcggcg gcattggtgg gagcgggggt tacagcgctg 420
gatccatgga tctnnnnnng catgcctgca gnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnnnnn nnnnnnnnnn nnnnnnnnnn nnnnnnnnnn nnnnnnnnnn nnnnnnnnnn 600
nnnnnnnnnn nnnnnnnnnn nnnnnnnnnn nnnnnnnnnn nnnnnnnnnn nnnnnnnnnn 660
nnnnnnnnnn nnnnnnnnnn nnnnnnnnnn nnnnnnnnnn nnnngatcgg cggggcctgg 720
cggcgcctcg ccgtcgcaga accaggcggt ggcgtacacc gtcgcctcgg tcggcccgta 780
gagattggcg atcccgaccg cagcaccacc gagaacgtcc ccgacgtggc cgaccagccc 840
gtcatcgtca acgcctgacc gcggtgcgga caggccgtgt cgcgaccggc cgtgcggaat 900
taagccggcc cgtaccctgt gaatagaggt ccgctgtgac acaagaatcc ctgttacttc 960
tcgaccgtat tgattcggat gattcctacg cgagcctgcg gaacgaccag gaattctggg 1020
agccgctggc ccgccgagcc ctggaggagc tcgggctgcc ggtgccgccg gtgctgcggg 1080
tgcccggcga gagcaccaac cccgtactgg tcggcgagcc cgacccggtg atcaagctgt 1140
tcggcgagca ctggtgcggt ccggagagcc tcgcgtcgga gtcggaggcg tacgcggtcc 1200
tggcggacgc cccggtgccg gtgccccgcc tcctcggccg cggcgagctg cggcccggca 1260
ccggagcctg gccgtggccc tacctggtga tgagccggat gaccggcacc acctggcggt 1320
ccgcgatgga cggcacgacc gaccggaacg cgctgctcgc cctggcccgc gaactcggcc 1380
gggtgctcgg ccggctgcac agggtgccgc tgaccgggaa caccgtgctc accccccatt 1440
ccgaggtctt cccggaactg ctgcgggaac gccgcgcggc gaccgtcgag gaccaccgcg 1500
ggtggggcta cctctcgccc cggctgctgg accgcctgga ggactggctg ccggacgtgg 1560
acacgctgct ggccggccgc gaaccccggt tcgtccacgg cgacctgcac gggaccaaca 1620
tcttcgtgga cctggccgcg accgaggtca ccgggatcgt cgacttcacc gacgtctatg 1680
cgggagactc ccgctacagc ctggtgcaac tgcatctcaa cgccttccgg ggcgaccgcg 1740
agatcctggc cgcgctgctc gacggggcgc agtggaagcg gaccgaggac ttcgcccgcg 1800

aactgctcgc cttcaccttc ctgcacgact tcgaggtgtt cgaggagacc ccgctggatc 1860
tctccggctt caccgatccg gaggaactgg cgcagttcct ctgggggccg ccggacaccg 1920
cccccggcgc ctgacgcccc ccaccgatct cgccgcgcag acaatggctg gctcagcggc 1980
gatgctgctg gagcggatgg agcaagacca gggtggcgcc aatggcgagc tgatggggct 2040
gcgcgtggac cttaccgcat cacggctgcg cgccgcggtt acctcgccgg gcggtacgac 2100
cgccgctgcg ctgcgggaac tcgaacgcgg cgggtttcgg atggctgtcg acgcggcggt 2160
tcaagccgcc aaaagccgct ctgagcagct cagaattaca ccggaatga 2209

<210> 3
<211> 2366
<212> DNA
<213> Mycobacterium tuberculosis

<220>
<221> misc_feature
<222> (1838)...(2090)
<223> unknown

<220>
<221> misc_feature
<222> (2103)...(2108)
<223> unknown

<400> 3
gtggcgctgt cagctgaggg ttcttcgggg ggatctcgcg ggggctcgcc gaaagcagaa 60
gccgcatcgg tgccgtcctg gccgcagatt ttggggcggt tgaccgacaa ccgcgatctg 120
gcgcggggcc aggctgcctg ggccatggac cagatcatga ccggcaatgc gcggccggcg 180
cagatcgccg ccttcgcggt ggcgatgacg atgaaggctc cgaccgccga cgaagtcggt 240
gagctcgctg gcgtcatgct cagccacgcg catccgttgc ccgccgatac ggtccccgac 300
gacgccgtcg acgtcgtcgg caccggtggc gacggagtca acacggtgaa cctatccacc 360
atggcggcga ttgtggtggc ggctgcgggt gtgccggtgg tcaagcacgg caaccgagcg 420
gcgtcctcgt tgtccggtgg cgccgacacg ctggaggcgc tgggggtgcg catcgacctg 480
ggacccgacc tggtcgcgcg cagcctcgcg gaggttggga tcgggttctg cttcgcgccg 540
cggttccatc cctcctaccg gcacgcggcc gccgggggcc gggggcggcg ccgggcggcc 600
cggggcgtca ggcgccgggg gcggtgtccg gcggccccca gaggaactgc gccagttcct 660
ccggatcggt gaagccggag agatccagcg gggtctcctc gaacacctcg aagtcgtgca 720
ggaaggtgaa ggcgagcagt tcgcgggcga agtcctcggt ccgcttccac tgcgccccgt 780
cgagcagcgc ggccaggatc tcgcggtcgc cccggaaggc gttgagatgc agttgcacca 840
ggctgtagcg ggagtctccc gcatagacgt cggtgaagtc gacgatcccg gtgacctcgg 900
tcgcggccag gtccacgaag atgttggtcc cgtgcaggtc gccgtggacg aaccggggtt 960
cgcggccggc cagcagcgtg tccacgtccg gcagccagtc ctccaggcgg tccagcagcc 1020
ggggcgagag gtagccccac ccgcggtggt cctcgacggt cgccgcgcgg cgttcccgca 1080
gcagttccgg gaagacctcg gaatgggggg tgagcacggt gttcccggtc agcggcaccc 1140

tgtgcagccg gccgagcacc cggccgagtt cgcgggccag ggcgagcagc gcgttccggt 1200
cggtcgtgcc gtccatcgcg gaccgccagg tggtgccggt catccggctc atcaccaggt 1260
agggccacgg ccaggctccg gtgccgggcc gcagctcgcc gcggccgagg aggcggggca 1320
ccggcaccgg ggcgtccgcc aggaccgcgt acgcctccga ctccgacgcg aggctctccg 1380
gaccgcacca gtgctcgccg aacagcttga tcaccgggtc gggctcgccg accagtacgg 1440
ggttggtgct ctcgccgggc acccgcagca ccggcggcac cggcagcccg agctcctcca 1500
gggctcggcg ggccagcggc tcccagaatt cctggtcgtt ccgcaggctc gcgtaggaat 1560
catccgaatc aatacggtcg agaagtaaca gggattcttg tgtcacagcg gacctctatt 1620
cacagggtac gggccggctt aattccgcac ggccggtcgc gacacggcct gtccgcaccg 1680
cggtcaggcg ttgacgatga cgggctggtc ggccacgtcg gggacgttct cggtggtgct 1740
gcggtcggga tcgccaatct ctacgggccg accgaggcga cggtgtacgc caccgcctgg 1800
ttctgcgacg gcgaggcgcc gccaggcccc gccgatcnnn nnnnnnnnnn nnnnnnnnnn 1860
nnnnnnnnnn nnnnnnnnnn nnnnnnnnnn nnnnnnnnnn nnnnnnnnnn nnnnnnnnnn 1920
nnnnnnnnnn nnnnnnnnnn nnnnnnnnnn nnnnnnnnnn nnnnnnnnnn nnnnnnnnnn 1980
nnnnnnnnnn nnnnnnnnnn nnnnnnnnnn nnnnnnnnnn nnnnnnnnnn nnnnnnnnnn 2040
nnnnnnnnnn nnnnnnnnnn nnnnnnnnnn nnnnnnnnnn nnnnnnnnnn ctgcaggcat 2100
gcnnnnnnag atccatggat atctagattt aaagatctgg taccgcggcc gcggtgcgcg 2160
cggtgttggg cggtgccagg ggcccggtcc gggacgctgt ggtgctcaac gccgccggtg 2220
cgatcgtcgc ccacgccgga ttatccagcc gtgctgaatg gctgccggcc tgggaggagg 2280
ggttgcggcg ggccagtgcg gcaatcgaca ccggtgcggc cgaacagttg ctcgcgcgat 2340
gggtgcggtt cggtcggcag atctga 2366

<210> 4
<211> 888
<212> DNA
<213> Mycobacterium tuberculosis

<400> 4
atgcttttcg gcatggcaag gatcgcgatt atcggcggcg gcagcatcgg tgaggcattg 60
ctgtcgggtc tgctgcgggc gggccggcag gtcaaagacc tggtagtggc cgagcggatg 120
cccgatcgcg ccaactacct ggcgcagacc tattcggtgt tggtgacgtc ggcggccgac 180
gcggtggaga acgcgacgtt cgtcgtcgtc gcggtcaaac cagccgacgt cgagccggtg 240
atcgcggatc tggcgaacgc gactgcggcg gccgaaaacg acagtgctga gcaggtgttc 300
gtcaccgtgg tagcgggcat cacgatcgcg tatttcgaat ccaagctacc ggctgggacg 360
ccagtggtgc gtgcgatgcc gaacgcggcg gcattggtgg gagcgggggt tacagcgctg 420
gccaaaggcc gctttgtcac cccgcaacag cttgaggagg tctcggcctt gttcgacgcg 480
gtcggcggcg tgctgaccgt tccggaatcg cagttggacg cggtgaccgc ggtgtccggc 540
tcgggtccgg cctatttctt tctgctggtc gaggccctgg tggatgccgg agtcggggtg 600
ggcttgagcc gtcaggtggc caccgatctc gccgcgcaga caatggctgg ctcagcggcg 660
atgctgctgg agcggatgga gcaagaccag ggtggcgcca atggcgagct gatggggctg 720

cgcgtggacc ttaccgcatc acggctgcgc gccgcggtta cctcgccggg cggtacgacc 780
gccgctgcgc tgcgggaact cgaacgcggc gggtttcgga tggctgtcga cgcggcggtt 840
caagccgcca aaagccgctc tgagcagctc agaattacac cggaatga 888